# EUROPEAN PATENT APPLICATION

(11) **EP 2 308 993 A1**
(43) Date of publication of application: **13.04.2011**
(21) Application number: 10179352.9
(22) Date of filing: 24.09.2010
(51) Int. Cl.: C12Q 1/04, C12Q 1/12

(54) **Bacteria analysis apparatus, bacteria analysis method and computer program product**

(30) Priority: 28.09.2009 JP 2009222645; 27.08.2010 JP 2010191297
(71) Applicant: SYSMEX CORPORATION, Kobe-shi, Hyogo 651-0073 (JP)
(72) Inventor: Wada, Atsushi, Hyogo 651-0073 (JP)
(74) Representative: HOFFMANN EITLE

(57) **Abstract**

A bacteria analysis apparatus comprising: a specimen preparation section for preparing a first measurement specimen from a sample by using a first enzyme; a detecting section for detecting bacteria included in the first measurement specimen; and an information processing section for outputting information for supporting determination or kind of bacteria included in the sample on the basis of the detection result of the first measurement specimen. A method and a computer program product is also disclosed.

## Description

### FIELD OF THE INVENTION

The present invention relates to a bacteria analysis apparatus, a bacteria analysis method and a computer program.

### BACKGROUND

In the past, as an apparatus for determining the kind of bacteria included in a sample, for example, an apparatus described in United States Patent Publication No. 2005/0079569 has been known.
In this apparatus, a particle measurement apparatus using a flow cytometer is used to determine the kind of bacteria included in a sample. In this apparatus, a first measurement specimen is prepared from a sample and a second measurement specimen is prepared from a sample and an alkaline solution. The first measurement specimen and the second measurement specimen are measured by the particle measurement apparatus. In addition, on the basis of the measurement result of the first measurement specimen and the measurement result of the second measurement specimen, whether bacteria are gram-positive bacteria or gram-negative bacteria is analyzed. In the above-mentioned United States Patent Publication No. 2005/0079569, it is described that an alkaline solution of about pH14 is used as the alkaline solution.

However, in the above-mentioned United States Patent Publication No. 2005/0079569, there is a problem in that it is necessary to introduce a highly corrosive strong alkaline solution of about pH14 into the apparatus.

### SUMMARY

The scope of the present invention is defined solely by the appended claims, and is not affected to any degree by the statements within this summary.
(1) A first aspect of the present invention is a bacteria analysis apparatus comprising:a specimen preparation section for preparing a first measurement specimen from a sample by using a first enzyme; a detecting section for detecting bacteria included in the first measurement specimen; and an information processing section for outputting information for supporting determination of kind of bacteria included in the sample on the basis of the detection result of the first measurement specimen. According to this configuration, even when a highly corrosive strong alkaline solution is not introduced into the apparatus, the information for supporting the determination of the kind of bacteria included in the sample can be output by using an enzyme (a first enzyme).
(2) The apparatus of (1), wherein the specimen preparation section prepares a second measurement specimen from the sample, the detecting section detects bacteria included in the first measurement specimen and bacteria included in the second measurement specimen, and the information processing section outputs information for supporting the determination of the kind of bacteria included in the sample on the basis of the detection result of the first measurement specimen and the detection result of the second measurement specimen. According to this configuration, the kind of bacteria included in the sample can be easily determined on the basis of the detection result of the first measurement specimen and the detection result of the second measurement specimen.
(3) The apparatus of (2), wherein the information processing section obtains degree of influence of the first enzyme on the bacteria included in the sample on the basis of the detection result of the first measurement specimen and the detection result of the second measurement specimen, and outputs information for supporting the determination of the kind of bacteria included in the sample on the basis of the degree of influence. If configured in this manner, by using as the first enzyme an enzyme which is known to have an influence on certain bacteria (react with certain bacteria), it can be estimated that the bacteria are included in a sample in which the influence of the first enzyme is recognized on the basis of the detection result of the first measurement specimen and the detection result of the second measurement specimen. The kind of the bacteria which are estimated can be output as information for supporting the determination of the kind of bacteria included in the sample.
(4) The apparatus of (3), wherein the information processing section obtains a value reflecting number of the bacteria included in the first measurement specimen and a value reflecting number of the bacteria included in the second measurement specimen on the basis of the detection result of the first measurement specimen and the detection result of the second measurement specimen, obtains the degree of influence of the first enzyme on the bacteria included in the sample on the basis of the value reflecting the number of bacteria of the first measurement specimen and the value reflecting the number of bacteria of the second measurement specimen, and outputs information for supporting the determination of the kind of bacteria included in the sample on the basis of the degree of influence. If configured in this manner, the degree of influence of the first enzyme on the bacteria included in the sample can be easily obtained on the basis of the value reflecting the number of bacteria of the first measurement specimen and the value reflecting the number of bacteria of the second measurement specimen.
(5) The apparatus of (4), wherein the information processing section creates a first scattergram relating to the bacteria included in the first measurement specimen and a second scattergram relating to the bacteria included in the second measurement specimen on the basis of the detection result of the first measurement specimen and the detection result of the second measurement specimen, and obtains the value reflecting the number of bacteria included in the first measurement specimen and the value reflecting the number of bacteria included in the second measurement specimen on the basis of the first scattergram and the second scattergram. If configured in this manner, values reflecting the number of bacteria included in the first measurement specimen and the second measurement specimen can be easily obtained on the basis of the first scattergram and the second scattergram.
(6) The apparatus of (3), wherein the information processing section creates a first scattergram relating to the bacteria included in the first measurement specimen and a second scattergram relating to the bacteria included in the second measurement specimen on the basis of the detection result of the first measurement specimen and the detection result of the second measurement specimen, obtains the degree of influence of the first enzyme on the bacteria included in the sample on the basis of the pattern of the first scattergram and the pattern of the second scattergram, and outputs the information for supporting the determination of the kind of bacteria included in the sample on the basis of the degree of influence. If configured in this manner, by comparing the pattern of the first scattergram with the pattern of the second scattergram (for example, a proportion, where the pattern of the first scattergram and the pattern of the second scattergram overlap, is calculated), the degree of influence of the first enzyme on the bacteria included in the sample can be easily obtained.
(7) The apparatus of any one of (1) to (6), wherein the information for supporting the determination of the kind of bacteria included in the sample at least includes a name of bacteria which may be included in the sample. If configured in this manner, since a tester can known a name of bacteria which may be included in the sample, it can be easily determined which bacteria are included in the sample.
(8) The apparatus of any one of (1) to (7), further comprising: a display section, wherein the information processing section performs a control operation so as to display information for supporting the determination of the kind of bacteria included in the sample on the display section. If configured in this manner, a tester can easily obtain the information for supporting the determination of the kind of bacteria included in the sample by viewing the display section.
(9) The apparatus of any one of (1) to (8), wherein the information for supporting the determination of the kind of bacteria included in the sample is information on whether or not the bacteria included in the sample are gram-positive bacteria. If configured in this manner, a tester can easily determine whether the bacteria included in the sample are gram-positive bacteria which are not Staphylococcus or gram-negative bacteria and gram-positive bacteria which are Staphylococcus on the basis of the output information.
(10) The apparatus of any one of (1) to (9), wherein the first enzyme is lysozyme, and the information for supporting the determination of the kind of bacteria included in the sample is information on whether or not the bacteria included in the sample are gram-positive bacteria which are not Staphylococcus or gram-negative bacteria and gram-positive bacteria which are Staphylococcus. If configured in this manner, lysozyme reacts with gram-positive bacteria which are not Staphylococcus, and thus by determining whether or not there is a reaction of bacteria by using lysozyme as the first enzyme, a tester can easily determine whether the bacteria included in the sample are gram-positive bacteria which are not Staphylococcus or gram-negative bacteria and gram-positive bacteria which are Staphylococcus.
(11) The apparatus of any one of (1) to (9), wherein the first enzyme is lysostaphin, and the information for supporting the determination of the kind of bacteria included in the sample is information on whether the bacteria included in the sample are bacteria which are Staphylococcus or bacteria which are not Staphylococcus. If configured in this manner, lysostaphin reacts with bacteria which are Staphylococcus, and thus by determining where or not there is a reaction of bacteria by using lysostaphin as the first enzyme, a tester can easily determine whether the bacteria included in the sample are bacteria which are Staphylococcus or bacteria which are not Staphylococcus.
(12) The apparatus of (2), wherein the specimen preparation section further prepares a third measurement specimen from the sample by using a second enzyme, the detecting section detects bacteria included in the first measurement specimen, bacteria included in the second measurement specimen and bacteria included in the third measurement specimen, and the information processing section outputs information for supporting the determination of the kind of bacteria included in the sample on the basis of the detection result of the first measurement specimen, the detection result of the second measurement specimen and the detection result of the third measurement specimen. If configured in this manner, the kind of bacteria included in the sample can be more accurately estimated by using the first enzyme and the second enzyme.
(13) The apparatus of (12), wherein the first enzyme is lysozyme and the second enzyme is lysostaphin, and the information processing section determines whether the bacteria included in the sample are bacteria which are Staphylococcus, gram-positive bacteria which are not Staphylococcus or gram-negative bacteria on the basis of the detection result of the first measurement specimen, the detection result of the second measurement specimen and the detection result of the third measurement specimen, and outputs information for supporting the determination of the kind of bacteria included in the sample on the basis of the determination result. If configured in this manner, lysozyme reacts with gram-positive bacteria which are not Staphylococcus and does not react with gram-negative bacteria, and lysostaphin reacts with bacteria (gram-positive bacteria) which are Staphylococcus and does not react with gram-negative bacteria, and thus by using lysozyme and lysostaphin, a tester can easily determine whether the bacteria included in the sample are bacteria which are Staphylococcus, gram-positive bacteria which are not Staphylococcus or gram-negative bacteria.
(14) The apparatus of Claim (10) or (13), wherein the concentration of lysozyme included in the measurement specimen is in the range of 2. 5 to 20 mg/mL. If configured in this manner, the reactivity between lysozyme and gram-positive bacteria which are not Staphylococcus increases, and thus the kind of bacteria included in a sample can be easily determined.
(15) The apparatus of (11) or (13), wherein the concentration of lysostaphin included in the measurement specimen is in the range of 0.5 to 100 µg/mL. If configured in this manner, the reactivity between lysostaphin and bacteria which are Staphylococcus increases, and thus the kind of bacteria included in a sample can be easily determined.
(16) A second aspect of the present invention is a bacteria analysis method comprising: preparing a first measurement specimen from a sample by using a first enzyme; detecting bacteria included in the first measurement specimen; and outputting information for supporting the determination of kind of bacteria included in the sample on the basis of the detection result of the first measurement specimen. Due to such configuration, even when a highly corrosive strong alkaline solution is not introduced into the apparatus, the information for supporting the determination of the kind of bacteria included in the sample can be output by using an enzyme (a first enzyme).
(17) A third aspect of the present invention is a computer program product comprising: a computer readable medium; and instructions, on the computer readable medium, adapted to enable a general purpose computer to perform operations, comprising: obtaining the detection result of bacteria included in a first measurement specimen which is prepared from a sample by using a first enzyme; and outputting information for supporting the determination of kind of bacteria included in the sample on the basis of the detection result of the bacteria of the first measurement specimen. Due to such configuration, even when a highly corrosive strong alkaline solution is not introduced into the apparatus, the information for supporting the determination of the kind of bacteria included in the sample can be output by using an enzyme (a first enzyme).

### BRIEF DESCRIPTION OF THE DRAWINGS

Fig. 1 is a perspective view showing the overall configuration of a bacteria analysis apparatus according to a first embodiment of the invention.
Fig. 2 is a block diagram showing an analysis section of the bacteria analysis apparatus according to the first embodiment shown in Fig. 1.
Fig. 3 is a diagram schematically showing a specimen preparation section and an optical detection section of the bacteria analysis apparatus according to the first embodiment shown in Fig. 1.
Fig. 4 is a diagram showing the structure of the optical detection section of the bacteria analysis apparatus according to the first embodiment shown in Fig. is.
Fig. 5 is a flowchart for explaining the analysis operation of the bacteria analysis apparatus according to the first embodiment shown in Fig. 1.
Fig. 6 is a flowchart for explaining a first interpretation process of the analysis operation of the bacteria analysis apparatus according to the first embodiment shown in Fig. 5.
Fig. 7 is a diagram showing a first interpretation result screen showing the interpretation result of the first interpretation process of the analysis operation of the bacteria analysis apparatus according to the first embodiment shown in Fig. 1.
Fig. 8 is a flowchart for explaining a second interpretation process of the analysis operation of the bacteria analysis apparatus according to the first embodiment shown in Fig. 5.
Fig. 9 is a diagram showing a comprehensive analysis result screen showing the interpretation result of a comprehensive analysis process of the analysis operation of the bacteria analysis apparatus according to the first embodiment shown in Fig. 1.
Fig. 10 is a flowchart for explaining the comprehensive analysis process of the analysis operation of the bacteria analysis apparatus according to the first embodiment shown in Fig. 1.
Fig. 11 is a diagram for explaining a bacteria kind determination principle using lysozyme.
Fig. 12 is a diagram showing a first interpretation result screen showing the interpretation result of a first interpretation process of the analysis operation of a bacteria analysis apparatus according to a modified example of the first embodiment shown in Fig. 1.
Fig. 13 is a diagram showing a comprehensive analysis result screen showing the interpretation result of a comprehensive analysis process of the analysis operation of the bacteria analysis apparatus according to the modified example of the first embodiment shown in Fig. 1.
Fig. 14 is a flowchart for explaining a comprehensive analysis process of the analysis operation of a bacteria analysis apparatus according to a second embodiment shown in Fig. 1.
Fig. 15 is a diagram for explaining a bacteria kind determination principle using lysostaphin.
Fig. 16 is a flowchart for explaining the analysis operation of a bacteria analysis apparatus according to a third embodiment of the invention.
Fig. 17 is a flowchart for explaining a second interpretation process of the analysis operation of the bacteria analysis apparatus according to the third embodiment of the invention.
Fig. 18 is a flowchart for explaining a comprehensive analysis process of the analysis operation of the bacteria analysis apparatus according to the third embodiment of the invention.
Fig. 19 is a graph showing the relationship between the concentration of lysozyme and the number of bacteria which are Enterococcus faecalis.
Fig. 20A is a graph showing the relationship between the concentration of lysostaphin and the number of bacteria which are Staphylococcus aureus.
Fig. 20B is a graph showing the relationship between the concentration of lysostaphin and the number of bacteria which are *S. sciuri.*

### DETAILED DESCRIPTION OF THE EMBODIMENT

Hereinafter, embodiments embodying the invention will be described with reference to drawings.

### (First Embodiment)

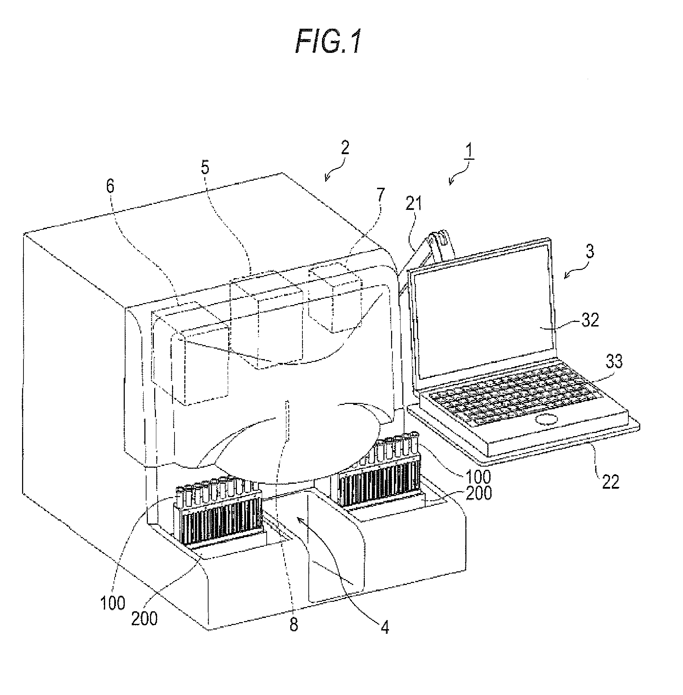

The overall configuration of a bacteria analysis apparatus 1 according to a first embodiment of the invention will be described with reference to Figs. 1 to 4.

As shown in Fig. 1, the bacteria analysis apparatus 1 according to the first embodiment includes a measurement section 2 for measuring a sample by flow cytometry and an analysis section 3 for analyzing the measurement result of the measurement section 2. The measurement section 2 includes a rack table 4 for transporting a sample rack 200 (test tube rack) which stores test tubes 100 containing a sample, a specimen preparation section 5 for preparing a measurement specimen from a sample or the like, an optical detection section 6 for detecting information of bacteria or formed elements in urine from a measurement specimen and a control section 7 for controlling the rack table 4, specimen preparation section 5, optical detection section 6 and the like. On the side surface of the chassis of the measurement section 2, a support 22 is attached via an arm 21. The analysis section 3 composed of a personal computer is installed on the support 22. In addition, the measurement section 2 and the analysis section 3 are connected by a LAN.

The rack table 4 has a function of transporting a sample rack 200 up to a predetermined sample suction position.

At the sample suction position, a syringe pump (not shown) suctions the sample (urine) in a test tube 100 by using a suction tube 8 and the sample is dispensed to a measurement specimen preparation section 51 to be described later.

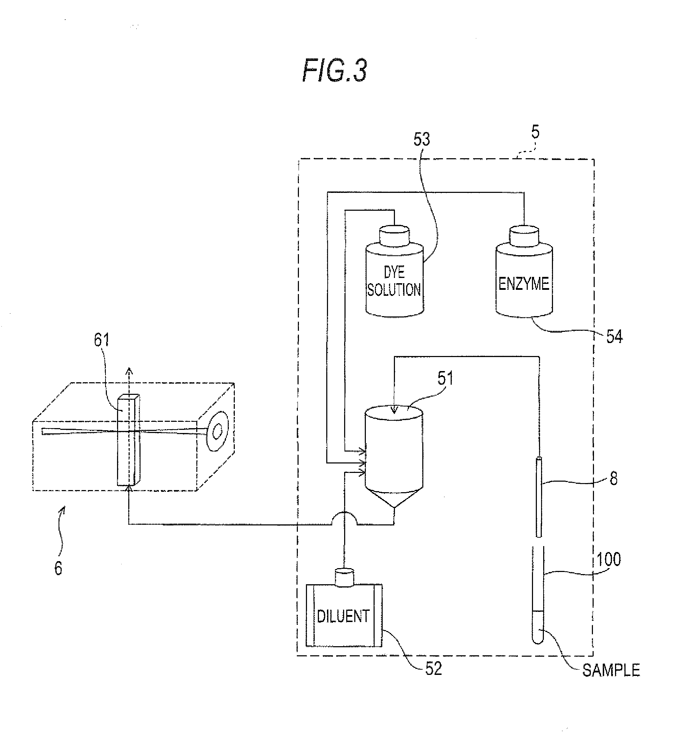

As shown in Fig. 3, the specimen preparation section 5 has a function of preparing a measurement specimen by mixing a sample with a predetermined liquid. In greater detail, the specimen preparation section 5 includes the measurement specimen preparation section 51, a diluent holding section 52, a dye solution holding section 53 and an enzyme holding section 54. In the first embodiment, the enzyme holding section 54 holds lysozyme which is a cell wall lytic enzyme. The specimen preparation section 5 can prepare a specimen for bacteria detection by mixing a dye solution with a diluent in the measurement specimen preparation section 51 in which a sample has been dispensed via the suction tube 8. In addition, the specimen preparation section 5 can prepare a first specimen for bacteria determination by mixing an enzyme (lysozyme.), a dye solution and a diluent in the measurement specimen preparation section 51 in which a sample has been dispensed. The measurement specimens (the specimen for bacteria detection and first specimen for bacteria determination) prepared in the specimen preparation section 5 are introduced to a flow cell 61 of the optical detection section 6 to be described later.

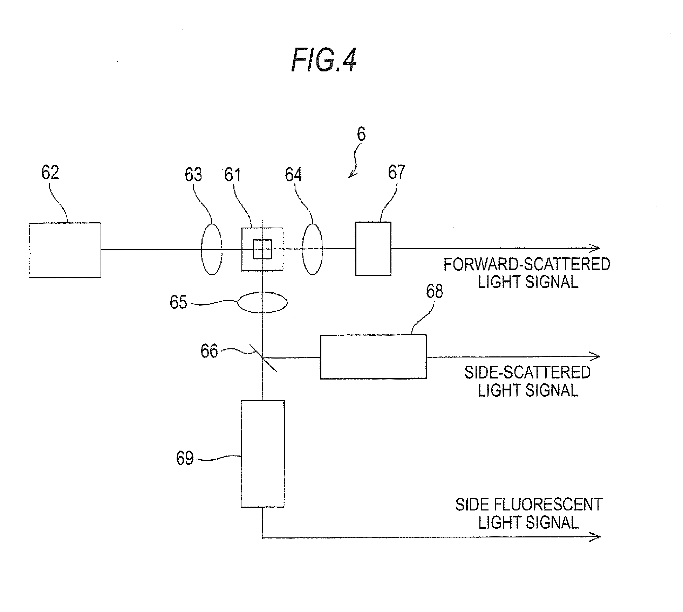

In the optical detection section 6, optical measurement is performed by flow cytometry. That is, in the optical detection section 6, a fine flow in which a measurement specimen is enclosed in a sheath liquid (not shown) is formed in the flow cell 61 and the fine flow is irradiated with laser light. As a specified configuration, as shown in Fig. 4, the optical detection section 6 includes a light source 62 composed of a semiconductor laser, a condenser lens 63 for focusing the laser light irradiated from the light source 62 on the fine flow of the flow cell 61, two condenser lenses 64 and 65, a dichroic mirror 66, a scattered light receiving section 67 composed of a photodiode, a scattered light receiving section 68 composed of a photomultiplier and a fluorescent light receiving section 69 composed of a photomultiplier.

The condenser lens 64 has a function of focusing forward-scattered light beams, from among light beams emitted from a measurement specimen (fine flow) in the flow cell 61 which are irradiated with laser light, on the scattered light receiving sections. 67. The condenser lens 65 has a function of focusing side-scattered light beams and side fluorescent light beams, from among light beams emitted from a measurement specimen in the flow cell 61 which is irradiated with laser light, on the dichroic mirror 66. The dichroic mirror 66 is configured to reflect side-scattered light beams toward the scattered light receiving section 68 and to transmit side fluorescent light beams through the fluorescent light receiving section 69.

The scattered light receiving section 67, scattered light receiving section 68 and fluorescent light receiving section 69 are configured to convert received light into electric signals. These electric signals reflect the shape, number and the like of bacteria included in a sample. That is, the optical detection section 6 can detect bacteria included in a measurement specimen (sample). These electric signals are transmitted to the analysis section 3 via the control section 7.

The operation of preparing a measurement specimen by the specimen preparation section 5, the operation of the measurement by the optical detection section 6 and the like are carried out automatically by operation of a magnetic valve, a driving section and the like (not shown) depending on the control of the control section 7 (microcomputer) of the measurement section 2.

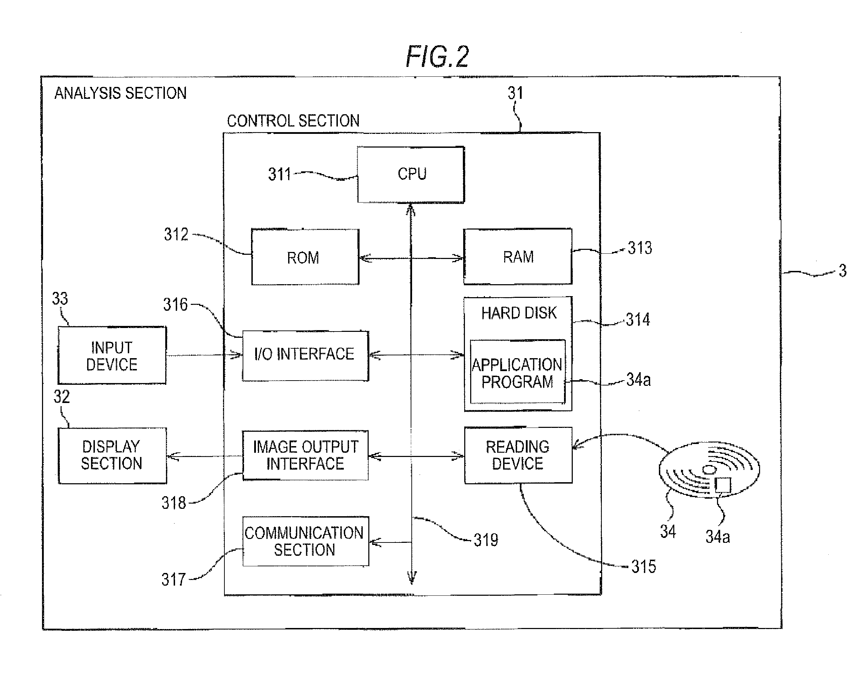

In addition, as shown in Figs. 1 and 2, the analysis section 3 is composed of a computer mainly including a control section 31 (see Fig. 2), a display section 32 and an input device 33.

As shown in Fig. 2, the control section 31 mainly includes a CPU 311, a ROM 312, a RAM 313, a hard disk 314, a reading device 315, an I/O interface 316, a communication section 317 and an image output interface 318. The CPU 311, ROM 312, RAM 313, hard disk 314, reading device 315, I/O interface 316, communication section 317 and image output interface 318 are connected by a bus 319.

The CPU 311 can execute computer programs stored in the ROM 312 and computer programs loaded to the RAM 313. When the CPU 311 executes a program 34a for analyzing the kind of bacteria, which will be described later, the computer functions as the analysis section 3.

Here, in the first embodiment, the CPU 311 of the analysis section 3 is configured to interpret the measurement data which is received from the control section 7 of the measurement section 2 via the communication section 317 and determine the kind of bacteria which may be included in a sample (urine), and to output information for supporting the determination of the kind of bacteria included in the sample on the basis of the determination result. In greater detail, the CPU 311 is configured to create a scattergram relating to the bacteria included in the sample by interpreting data from the measurement section 2. In addition, the CPU 311 is configured to calculate a numerical value corresponding to the number of bacteria included in a sample on the basis of the scattergram. The CPU 311 is configured to obtain the degree of influence of an enzyme with respect to bacteria included in a sample on the basis of the detection result (a scattergram, a numerical value corresponding to the number of bacteria and the like) of a specimen for bacteria detection which is prepared from the sample and the detection result of a first specimen for bacteria determination which is prepared from the sample and enzyme (lysozyme) and to output on the display section 32 information for supporting the determination of the kind of bacteria included in the sample on the basis of the degree of influence.

The ROM 312 is composed of a mask ROM, a PROM, an EPROM, an EEPROM or the like, and computer programs which are executed by the CPU 311 and data which are used in the execution of the programs are recorded therein.

The RAM 313 is composed of a SRAM, a DRAM or the like. The RAM 313 is used to read computer programs which are recorded in the ROM 312 and the hard disk 314. In addition, the RAM is used as a work area of the CPU 311 when these computer programs are executed.

In the hard disk 314, various computer programs for execution by the CPU 311, such as an operating system and an application program, and data which are used to execute the computer programs, are installed. The program for analyzing the kind of bacteria according to the first embodiment is also installed in the hard disk 314.

The reading device 315 is composed of a flexible disk drive, a CD-ROM drive, a DVD-ROM drive or the like and can read computer programs or data which are recorded in a portable recording medium 34. In addition, the program 34a for analyzing the kind of bacteria is stored in the portable recording medium 34 and the computer can read the program 34a for analyzing the kind of bacteria from the portable recording medium 34 and install the program in the hard disk 314.

The above-mentioned program 34a for analyzing the kind of bacteria is provided by the portable recording medium 34 and can be also provided from an external device, which is connected to the computer by an electric communication line (which may be wired or wireless) to communicate therewith, through the electric communication line. For example, the program 34a for analyzing the kind of bacteria is stored in the hard disk 314 of a server computer on the internet and the computer accesses the server computer to download the program 34a for analyzing the kind of bacteria and to install the program in the hard disk 314.

Further, in the hard disk 314, for example, an operating system is installed for providing a graphical user interface environment, such as Windows (registered trade name) which is made and distributed by Microsoft Corporation in America. In the following description, the program 34a for analyzing the kind of bacteria operates on the above-mentioned operating system.

The I/O interface 316 is composed of, for example, a serial interface such as USB, IEEE1394 or RS-232C, a parallel interface such as SCSI, IDE or IEEE1284, and an analog interface including a D/A converter and an A/D converter. The input device 33 is connected to the I/O interface 316 and a user uses the input device 33 so as to input data to the computer. Accordingly, the user can issue an instruction (input of a measurement order) to the analysis section 3 to perform the measurement by the measurement section 2.

For example, the communication section 317 is an Ethernet (registered trade name) interface. The analysis section 3 is configured to receive measurement data from the measurement section 2 via the communication section 317. In addition, the analysis section 3 can transmit a control signal to the measurement section 2 via the communication section 317.

The image output interface 318 is connected to the display section 32 composed of an LCD or a CRT so as to output to the display section 32 a picture signal corresponding to image data provided from the CPU 311. The display section 32 is configured to display an image (screen) in accordance with an input picture signal.

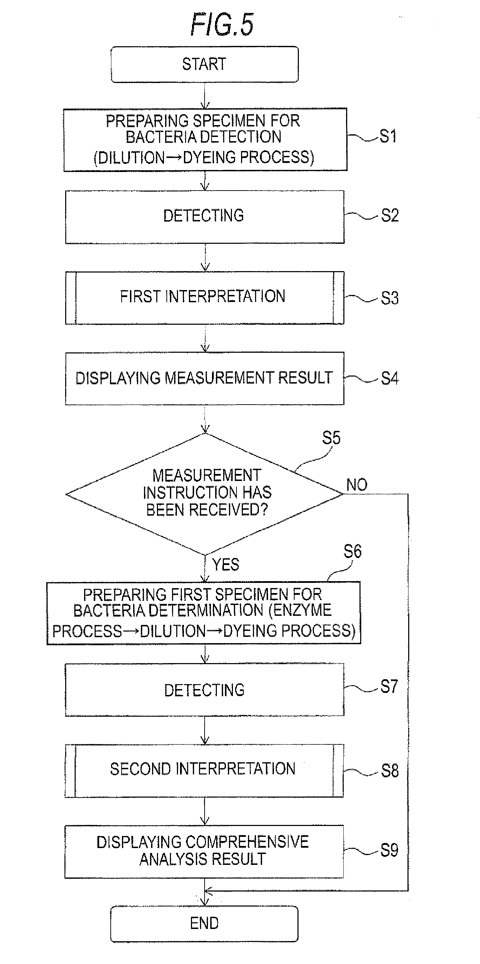

Next, the analysis operation of the bacteria analysis apparatus 1 according to the first embodiment will be described with reference to Figs. 5 to 10.

First, a tester issues an analysis instruction to the analysis section 3. Accordingly, the analysis is started in the measurement section 2. In greater detail, in Step S1, the control section 7 controls the specimen preparation section 5 to prepare a specimen for bacteria detection. That is, by mixing a specimen (urine), a diluent and a dye solution, a specimen for bacteria detection is prepared. After that, the prepared specimen for bacteria detection is transported to the optical detection section 6.

Next, in Step S2, the control section 7 measures the specimen for bacteria detection. In greater detail, the specimen for bacteria detection is made to flow to the flow cell 61 so as to form a fine flow and the flow (sample) is irradiated with laser light from the light source 62. Forward-scattered light beams, side-scattered light beams and side fluorescent light beams from the sample irradiated with laser light are received by the scattered light receiving section 67, scattered light receiving section 68 and fluorescent light receiving section 69, respectively. In the respective scattered light receiving section 67, scattered light receiving section 68 and fluorescent light receiving section 69, the forward-scattered light beams, side-scattered light beams and side fluorescent light beams are converted into electric signals. These electric signals are converted into digital data in the control section 7 and subjected to predetermined waveform processing. The measurement result (data corresponding to the forward-scattered light beams, data corresponding to the side-scattered light beams and data corresponding to the side fluorescent light beams) of the specimen for bacteria detection prepared by such processes is transmitted to the analysis section 3.

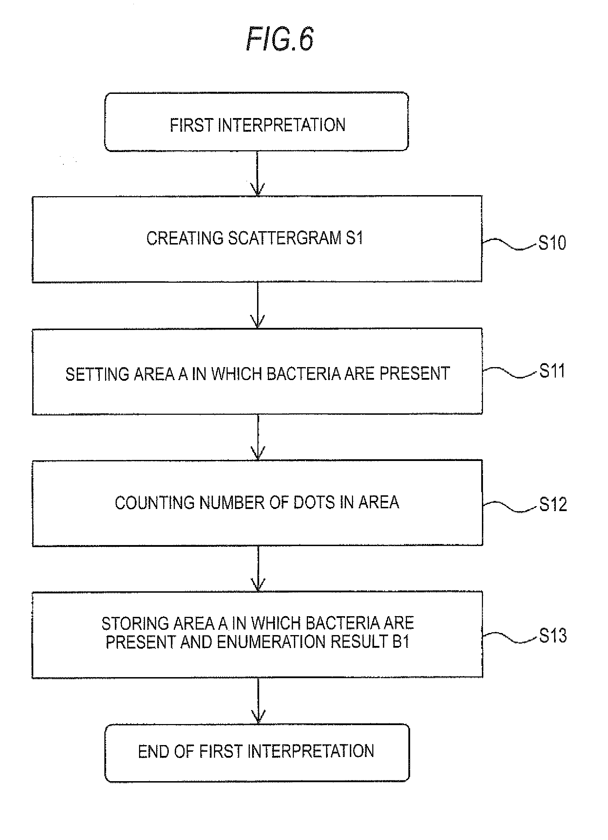

Next, in Step S3, the CPU 311 of the analysis section 3 performs a first interpretation process. That is, as shown in Fig. 6, the CPU 311 creates a scattergram S1 of the specimen for bacteria detection on the basis of the data corresponding to the forward-scattered light beams and the data corresponding to the side fluorescent light beams in Step S10. The CPU 311 sets an area A where bacteria are present in the scattergram S1 in Step S11 and counts the number of dots included in the area A in Step S12 to obtain an enumeration result B1 of the specimen for bacteria detection. The number of dots (enumeration result B1) is a value reflecting the number of bacteria included in the sample. In Step S13, the CPU 311 stores the area A and the enumeration result B1 on the hard disk 314.

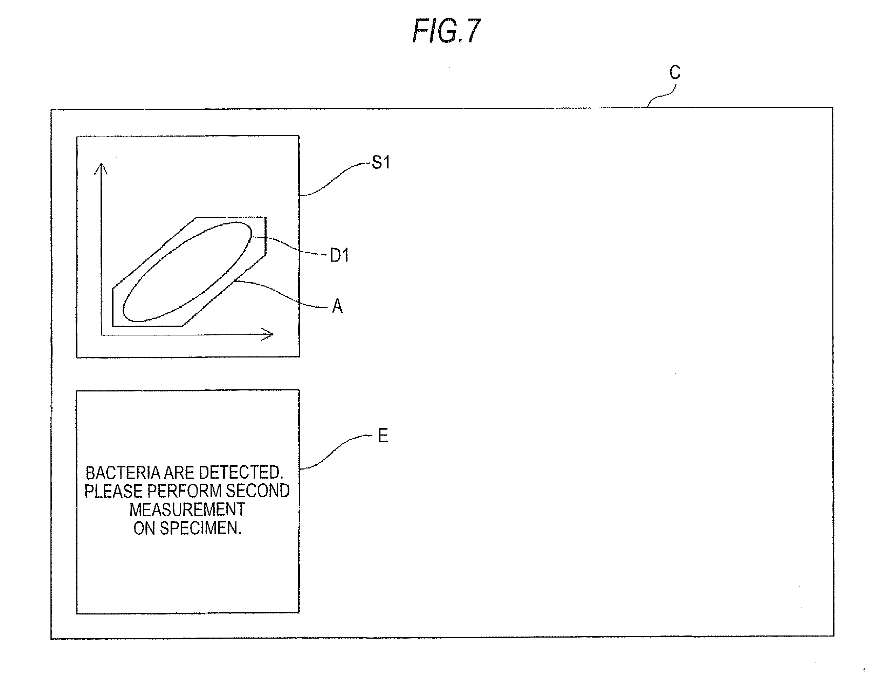

In Step S4, the CPU 311 displays a first interpretation result on the display section 32 of the analysis section 3 (personal computer). In greater detail, as shown in Fig. 7, a first interpretation result screen C including the scattergram S1 of the specimen for bacteria detection created in Step S10 and the enumeration result B1 of the specimen for bacteria detection is displayed on the display section 32. In the scattergram S1 of the first interpretation result screen C, a dot group D1 showing a bacteria distribution situation and the area A surrounding the group D1 are shown. In addition, when bacteria are detected as a result of the analysis, the effect thereof and a message E for prompting the execution of the measurement for determining the kind of the detected bacteria are displayed along with the first interpretation result screen C.

By referring to the first interpretation result screen C, the tester decides whether or not to perform the measurement for determining the kind of the detected bacteria. When performing second interpretation, the tester issues an instruction to the bacteria analysis apparatus 1 to perform the measurement by pressing a measurement instruction button (not shown) on the screen displayed on the display section 32 of the analysis section 3. In addition, in Step S5 of Fig. 5, the CPU 311 determines whether the measurement instruction button has been pressed or not. When the measurement instruction button has not been pressed, the analysis process of the bacteria analysis apparatus 1 ends. When the measurement instruction button has been pressed, the CPU 311 instructs the measurement section 2 to start the measurement.

When the instruction for the measurement is issued, the control section 7 of the measurement section 2 controls the specimen preparation section 5 in Step S6 to prepare a first specimen for bacteria determination. That is, by mixing an enzyme (lysozyme) with the sample (urine), diluent and dye solution, a first specimen for bacteria determination is prepared. After that, the prepared first specimen for bacteria determination is transported to the optical detection section 6.

Next, in Step S7, the control section 7 measures the first specimen for bacteria determination as in Step S2. The measurement result (data corresponding to forward-scattered light beams, data corresponding to side-scattered light beams and data corresponding to side fluorescent light beams) of the first specimen for bacteria determination is transmitted to the analysis section 3.

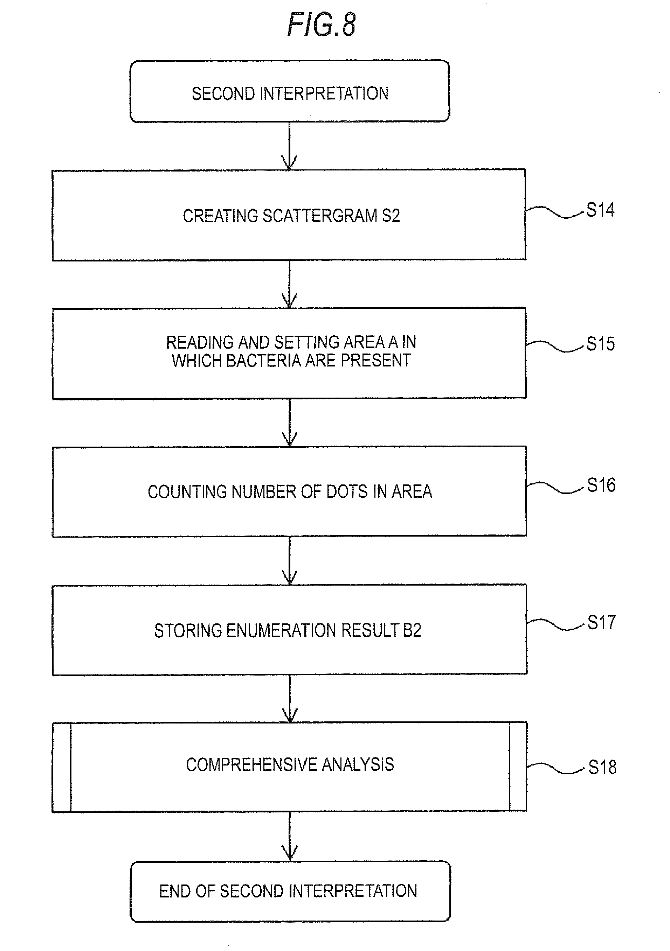

Next, in Step S8, the CPU 311 interprets the measurement data of the first specimen for bacteria determination (second interpretation process). That is, as shown in Fig. 8, in Step S14, a scattergram S2 of the first specimen for bacteria determination is created on the basis of the data corresponding to the forward-scattered light beams and the data corresponding to the side fluorescent light beams. In Step S15, the area A set in Step S11 (see Fig. 6) is read and set in the scattergram S2. In Step S16, the number of dots included in the area A in the scattergram S2 is counted to obtain an enumeration result B2 of the first specimen for bacteria determination. The number of dots (enumeration result B2) is a value reflecting the number of bacteria included in the first specimen for bacteria determination. In Step S17, the enumeration result B2 is stored on the hard disk 314.

After that, in Step S18, a comprehensive analysis is performed.

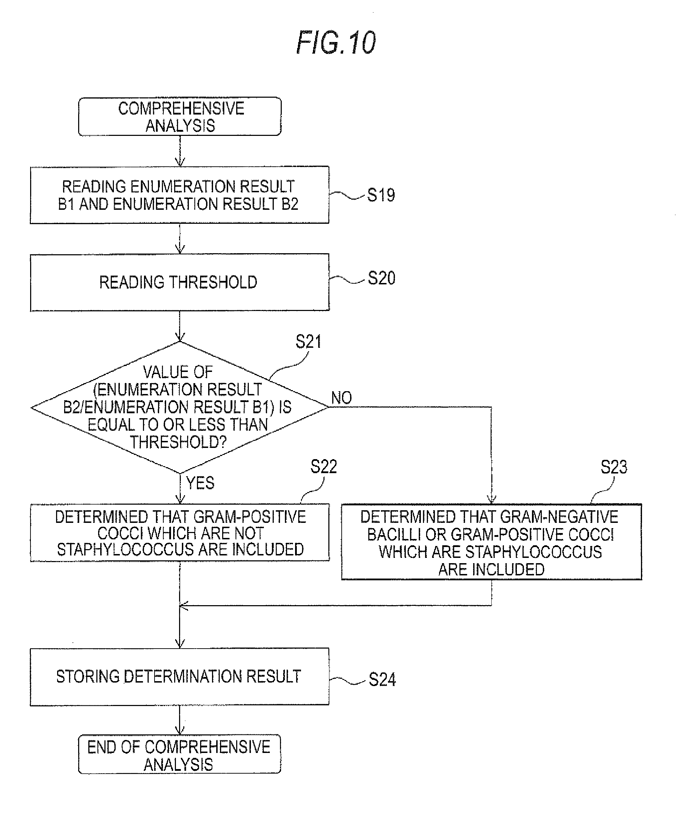

In the comprehensive analysis, first, as shown in Fig. 10, the enumeration result B1 of the specimen for bacteria detection and the enumeration result B2 of the first specimen for bacteria determination stored in the hard disk 314 are read inStepS19. Next, in Step S20, a predetermined threshold which is set in advance is read from the hard disk 314.

In Step S21, the CPU 311 determines whether or not the value of (enumeration result B2/enumeration result B1) is equal to or less than the threshold. The (enumeration result B2/enumeration result B1) is a value indicating the degree of influence of the lysozyme on the bacteria included in the sample. By comparing the degree of influence with the threshold, the degree of influence of the lysozyme is quantitatively determined. When the value of (enumeration result B2/enumeration result B1) is equal to or less than the threshold, in Step S22, the CPU 311 determines that the bacteria included in the sample are gram-positive cocci which are not Staphylococcus. When the value of (enumeration result B2/enumeration result B1) is greater than the threshold, in Step S23, the CPU 311 determines that the bacteria included in the sample are gram-negative bacilli or gram-positive cocci which are Staphylococcus.

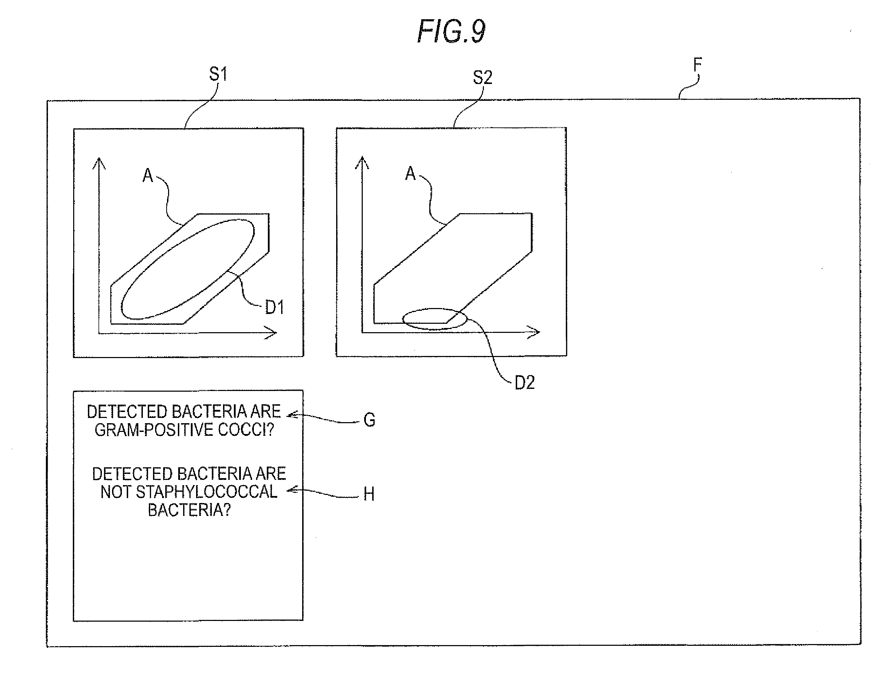

After the determination result is stored in Step S24, a comprehensive analysis result screen F showing the result of the comprehensive analysis is displayed on the display section 32 in Step S9 (see Fig. 5). As shown in Fig. 9, in the comprehensive analysis result screen F, the scattergram S1 displayed in the first interpretation result screen C in Step S4, the scattergram S2 created in Step S14 and information based on the determination result of the comprehensive analysis in the above-mentioned Step S18 (Steps S19 to S24) are displayed. In the scattergram S2, a dot group D2 and the area A which is the same as in the scattergram S1 are shown. In Fig. 9, an example is shown in which the enumeration result B2 of the first specimen for bacteria determination is remarkably smaller than the enumeration result B1 of the specimen for bacteria detection and the value of (enumeration result B2 of the first specimen for bacteria detection/enumeration result B1 of the specimen for bacteria detection) is equal to or less than a threshold. In this case, in Step S22 (see Fig. 10), the bacteria included in the sample are determined as gram-positive cocci which are not Staphylococcus, and in the comprehensive analysis result screen F, a message G suggesting that the detected bacteria are gram-positive cocci and a message H suggesting that the detected bacteria are not staphylococcal bacteria are displayed. By referring to the messages G and H, the kind of bacteria included in the sample can be determined.

Next, a bacteria kind determination principle using lysozyme will be described.

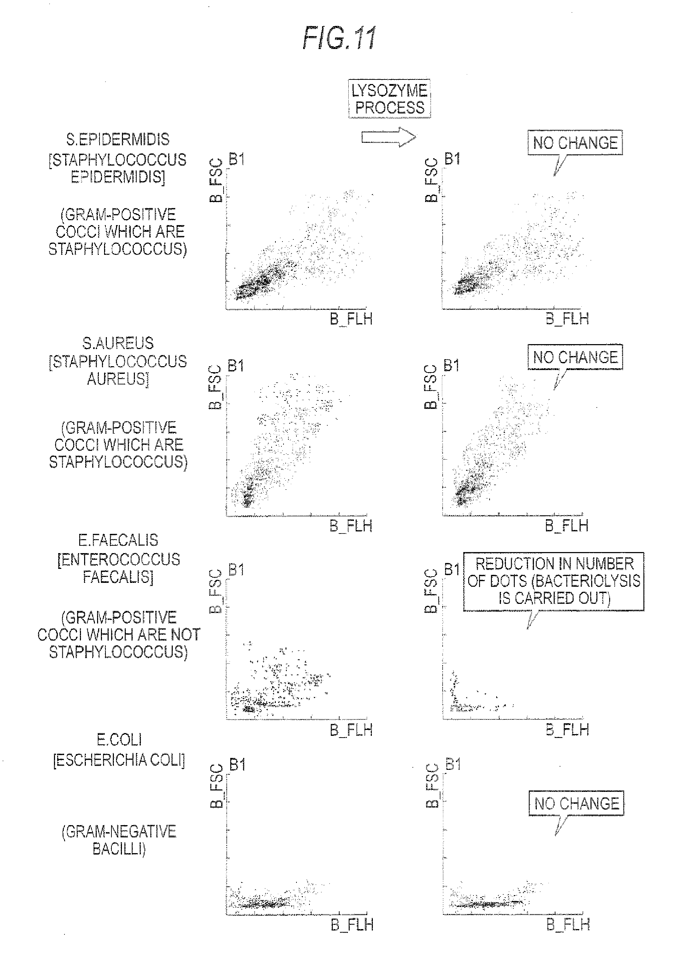

Regarding a sample including at least one of the following four kinds of bacteria, a specimen for bacteria detection is prepared from the sample, diluent and dye solution and a first specimen for bacteria determination is prepared from the sample, diluent, dye solution and lysozyme as in the analysis of the bacteria analysis apparatus 1 according to the first embodiment so as to be measured by flow cytometry, and scattergrams created by the measurement are shown in Fig. 11. The four kinds of bacteria are Staphylococcus epidermidis (S. epidermidis), Staphylococcus aureus (S. aureus), Enterococcus faecalis (E. faecalis) and Escherichia coli (E. coli). These four kinds of bacteria are known as bacteria which cause urinary tract infection. Particularly, an uncomplicated urinary tract infection is usually caused by any one of the four kinds of bacteria. Staphylococcus epidermidis and Staphylococcus aureus are gram-positive bacteria which are Staphylococcus. Enterococcus faecalis is gram-positive bacteria of Enterococcus (which are not Staphylococcus). Escherichia coli are gram-negative bacilli.

As shown in Fig. 11, regarding Staphylococcus and Escherichia coli, a difference in the number of dots between the measurement result of the specimen for bacteria detection and the measurement result of the first specimen for bacteria determination cannot be shown. On the other hand, regarding Enterococcus faecalis, the number of dots of the measurement result of the first specimen for bacteria determination is remarkably smaller than in the measurement result of the specimen for bacteria detection. That is, it is found that lysozyme reacts only with Enterococcus faecalis, not with Staphylococcus and Escherichia coli, and carries out the bacteriolysis.

In this manner, a sample is divided into two to prepare a specimen for bacteria detection by using one sample without the addition of lysozyme and prepare a first specimen for bacteria determination by using the other sample with the addition of lysozyme. Whether or not the bacteria included in the sample are Enterococcus faecalis can be determined on the basis of the measurement results of the two measurement specimens.

As the concentration of lysozyme included in the first specimen for bacteria determination, it is desirable that in gram-positive bacteria which are not Staphylococcus such as Enterococcus faecalis, a concentration is appropriately set at which a difference in the number of dots between the measurement result of the specimen for bacteria detection and the measurement result of the first specimen for bacteria determination can be recognized. The concentration of lysozyme in the first specimen for bacteria determination is preferably in the range of 2.5 to 20 mg/mL. When the concentration of lysozyme is equal to or higher than 2.5 mg/mL, the difference in the number of dots between the measurement result of the specimen for bacteria detection and the measurement result of the first specimen for bacteria determination can be easily recognized. When the concentration of lysozyme is equal to or lower than 20 mg/mL, lysozyme can be easily dissolved in the first specimen for bacteria determination and thus the first specimen for bacteria determination can be easily prepared.

In this manner, a sample is divided into two aliquots to prepare a specimen for bacteria detection in one aliquot without addition of lysozyme and prepare a first specimen for bacteria determination in the other aliquot with addition of lysozyme and it can be determined whether or not the bacteria included in the sample are Enterococcus faecalis on the basis of the measurement results of the two measurement specimens.

In addition, in the configuration using lysozyme according to the first embodiment, it is difficult to perform a determination of bacteria which are not Enterococcus faecalis (determination of the bacteria analysis apparatus between Staphylococcus and gram-negative bacilli). However, by using a difference between the pattern of the scattergram of Staphylococcus and the pattern of the scattergram of gram-negative bacilli, Staphylococcus and gram-negative bacilli can be determined. That is, Staphylococcus and gram-negative bacilli can be determined from the fact that in the pattern of Staphylococcus, the distribution state of dots has a steep slope (the pattern rises), and in the pattern of gram-negative bacilli, the distribution state of dots has a gradual slope (the pattern is flat). In this manner, on the basis of the analysis result of the bacteria analysis apparatus 1 according to the first embodiment and the shape of the patterns of the scattergrams, Staphylococcus, Enterococcus faecalis and gram-negative bacilli can be determined.

In the first embodiment, as mentioned above, by providing the analysis section 3 which outputs information for supporting the determination of the kind of bacteria included in a sample on the basis of the detection result of a specimen for bacteria detection prepared from the sample and the detection result of a first specimen for bacteria determination prepared from the sample and lysozyme, information for supporting the determination of the kind of bacteria included in a sample can be output by using an enzyme (lysozyme) with a low level of corrosion without introduction of a highly corrosive strong alkaline solution to the bacteria analysis apparatus 1.

In the first embodiment, as mentioned above, the analysis section 3 obtains the degree of influence of lysozyme on the bacteria included in a sample on the basis of the detection result of a specimen for bacteria detection and the detection result of a first specimen for bacteria determination, and outputs information for supporting the determination of the kind of bacteria included in the sample on the basis of the degree of influence. Due to such configuration, by using lysozyme which is known to react with Enterococcus faecalis (gram-positive bacteria which are not Staphylococcus), it can be estimated that a sample in which the influence of lysozyme is recognized on the basis of the detection result of a specimen for bacteria detection and the detection result of a first specimen for bacteria determination includes the bacteria (Enterococcus faecalis). The kind of the estimated bacteria can be output as information for supporting the determination of the kind of bacteria included in the sample.

In the first embodiment, as mentioned above, the degree of influence of lysozyme on the bacteria included in a sample is obtained on the basis of the value reflecting the number of bacteria of a specimen for bacteria detection and the value reflecting the number of bacteria of a first specimen for bacteria determination, and outputs information for supporting the determination of the kind of bacteria included in the sample on the basis of the degree of influence. Due to such configuration, the degree of influence of lysozyme on the bacteria included in a sample can be easily obtained on the basis of the value reflecting the number of bacteria of a specimen for bacteria detection and the value reflecting the number of bacteria of a first specimen for bacteria determination.

In the first embodiment, as mentioned above, the analysis section 3 creates the scattergram S1 relating to the bacteria included in a specimen for bacteria detection and the scattergram S2 relating to the bacteria included in a first specimen for bacteria determination on the basis of the detection result of the specimen for bacteria detection and the detection result of the first specimen for bacteria determination, and obtains values (enumeration results B1 and B2) reflecting the number of bacteria included in the specimen for bacteria detection and the first specimen for bacteria determination on the basis of the scattergram S1 and the scattergram S2. Due to such configuration, values reflecting the number of bacteria included in a specimen for bacteria detection and a first specimen for bacteria determination can be easily obtained on the basis of the scattergram S1 and the scattergram S2.

In the first embodiment, as mentioned above, as information for supporting the determination of the kind of bacteria included in a sample, the name of bacteria which may be included in the sample is displayed on the display section 32, and thus a tester can know the name of bacteria which may be included in the sample. Accordingly, it is possible to easily determine which bacteria are included in the sample.

In the first embodiment, as mentioned above, lysozyme reacts with gram-positive bacteria which are not Staphylococcus. Accordingly, by using lysozyme, a tester can easily determine whether the bacteria included in a sample are gram-positive bacteria which are not Staphylococcus or gram-negative bacteria and gram-positive bacteria which are Staphylococcus.

In the first embodiment, as mentioned above, by analyzing urine by using the bacteria analysis apparatus 1, the kind of bacteria included in the urine can be easily determined.

In the first embodiment, it is possible to determine whether or not staphylococcal bacteria are included in a sample. Staphylococcal bacteria may have drug resistance with respect to certain drugs. For example, when administering methicillin, staphylococcal bacteria mutate into methicillin-resistant Staphylococcus aureus in some cases. Accordingly, a determination of whether or not staphylococcal bacteria are included in a sample is important in the clinical tests. In the first embodiment, by referring to the messages G and H of the comprehensive analysis result screen F of the bacteria analysis apparatus 1, it can be determined whether or not staphylococcal bacteria are included in a sample on the basis of the pattern (dot group D1) of the scattergram S1 of a specimen for bacteria detection and the pattern (dot group D2) of the scattergram S2 of a first specimen for bacteria determination.

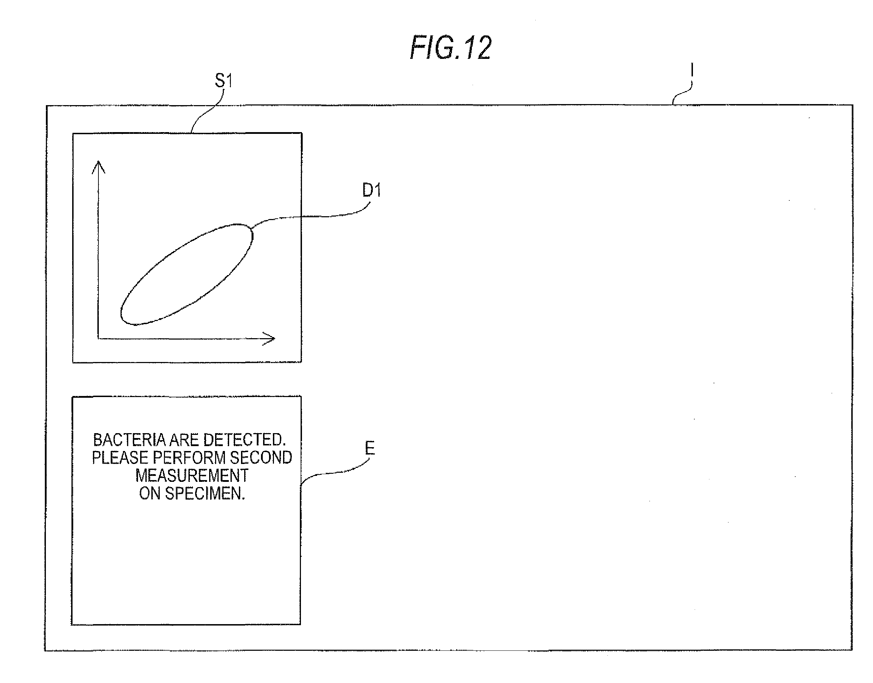

In the above-mentioned first embodiment, an example has been shown in which the number of dots included in the area A in each of the scattergram S1 of a specimen for bacteria detection and the scattergram S2 of a first specimens for bacteria determination are counted. However, the invention is not limited thereto. As in a first interpretation result screen I according to a modified example of the first embodiment shown in Fig. 12, the number of all of the dots in a scattergram may be counted without defining the area A.

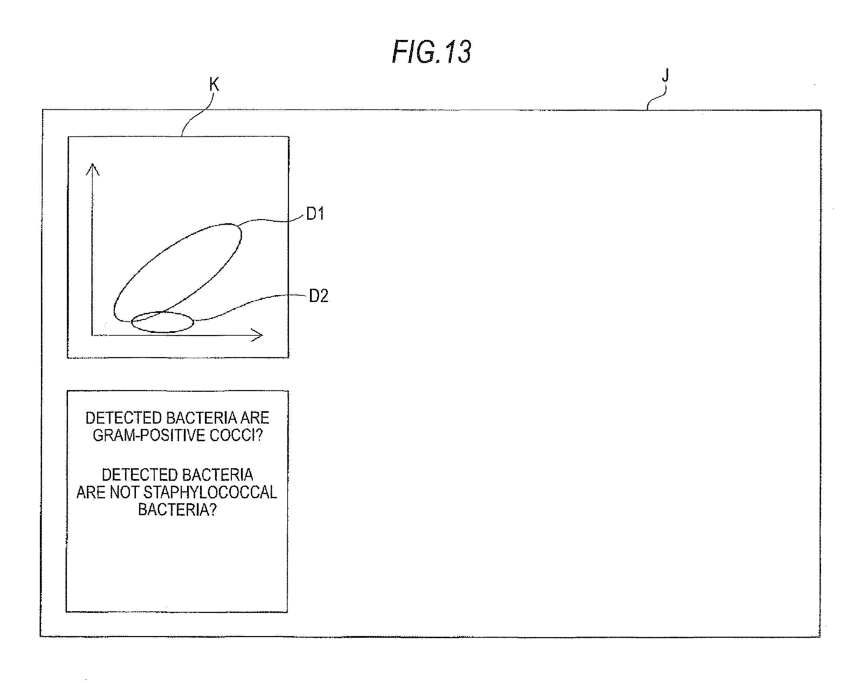

In the above-mentioned first embodiment, an example has been shown in which the scattergram S1 of a specimen for bacteria detection and the scattergram S2 of a first specimen for bacteria determination are displayed side by side in the comprehensive analysis result screen F. However, the invention is not limited thereto. As in a comprehensive analysis result screen J according to the modified example of the first embodiment shown in Fig. 13, the pattern (dot group D1) of the scattergram S1 of a specimen for bacteria detection and the pattern (dot group D2) of the scattergram S2 of a first specimen for bacteria determination may overlap with each other and be displayed in a scattergram K. In this manner, a tester can easily recognize a change in the pattern of the scattergram S2 of the first specimen for bacteria determination with respect to the scattergram S1 of the specimen for bacteria measurement.

In the above-mentioned first embodiment, an example has been shown in which the kind of bacteria is determined by comparing a ratio of the dot count result with a predetermined threshold. However, the invention is not limited thereto. The determination may be carried out by comparing a proportion of the area where the pattern (dot group D1) of the scattergram of a specimen for bacteria measurement and the pattern (dot group D2) of the scattergram of a first specimen for bacteria determination overlap with each other with a predetermined threshold which is set in advance with respect to the overlapping proportion. The overlapping proportion of the patterns of the scattergrams is a value indicating the degree of influence of lysozyme on the bacteria included in a sample. When the degree of influence of lysozyme is quantified using the amount of pattern overlapping in this manner, it is not necessary to count the number of dots of the scattergram in the determination of the kind of bacteria.

In the above-mentioned first embodiment, an example of the message in the comprehensive analysis result screen has been shown. However, other messages may be shown. For example, when it is determined that gram-positive bacteria (Enterococcus faecalis) which are not Staphylococcus are included, a message displaying the name of bacteria, such as "Staphylococcal bacteria are not included in the sample. Streptococcus may be included in the sample." may be shown, or a message displaying the number of bacteria, such as the number of staphylococcal bacteria included in the sample: 0" may be shown.
In addition, when it is determined that gram-negative bacilli or gram-positive cocci which are Staphylococcus are included, a message displaying the kind of bacteria, such as "Gram-negative bacteria may be included in the sample", a message displaying the name of bacteria, such as "Staphylococcal bacteria or Escherichia coli, may be included in the sample", or a message displaying the number of bacteria, such as "The number of staphylococcal bacteria included in the sample: measurement failure" may be shown.

### (Second Embodiment)

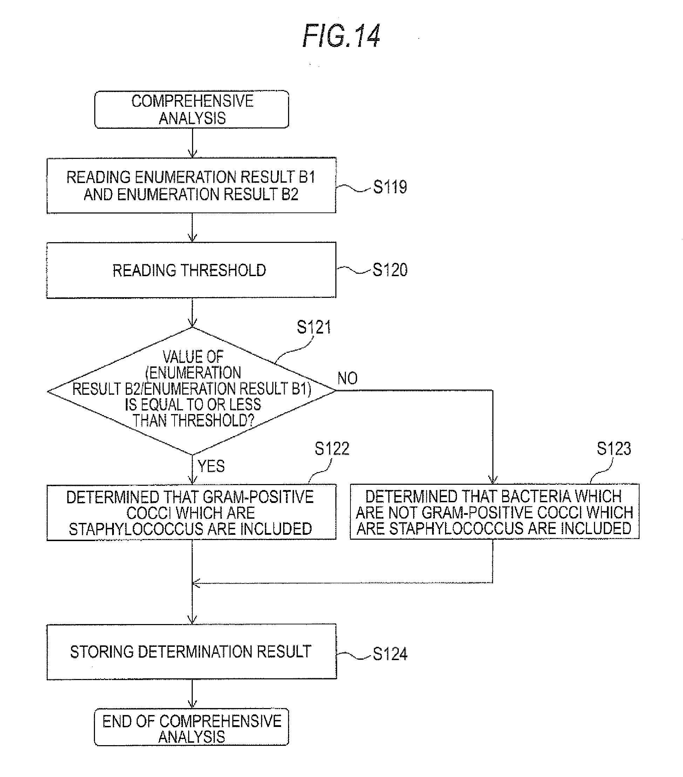

Next, a bacteria analysis apparatus 1 according to a second embodiment of the invention will be described with reference to Fig. 14. In the second embodiment, unlike the above-mentioned first embodiment in which lysozyme is used, an example using lysostaphin will be described. Since the structure of the bacteria analysis apparatus 1 according to the second embodiment is the same as in the above-mentioned first embodiment, except for an enzyme to be used and an interpretation process of the analysis section 3, the description of the structure of the bacteria analysis apparatus 1 will be omitted. Since the analysis flow and the interpretation flow are also the same as in the first embodiment, except for a comprehensive analysis process, the description thereof will be omitted.

In a comprehensive analysis process according to the second embodiment, in Steps S119 and S120 shown in Fig. 14, the same process is performed as in Steps S19 and S20 of Fig. 10. In Step S121, the CPU 311 determines whether or not the value of (enumeration result B2/enumeration result B1) is equal to or less than a threshold. When the value of (enumeration result B2/enumeration result B1 is equal to or less than a threshold, the CPU 311 determines that the bacteria included in a sample are gram-positive cocci which are Staphylococcus in Step S122. When the value of (enumeration result B2/enumeration result B1) is greater than the threshold, the CPU 311 determines that the bacteria included in a sample are bacteria which are not gram-positive cocci which are Staphylococcus in Step S123. In Step S124, after the storage of the determination result, the comprehensive analysis result is displayed on the comprehensive analysis result screen of the display section 32.

In the above-mentioned second embodiment, in the comprehensive analysis result screen, a scattergram of the detection result of a specimen for bacteria detection, a scattergram of the detection result of a first specimen for bacteria determination and information based on the determination result of the comprehensive analysis inS122and S123 are displayed as in the above-mentioned first embodiment. In greater detail, when it is determined that the bacteria included in a sample are gram-positive bacteria which are Staphylococcus, a message such as "Staphylococcal bacteria are included in the sample" or "The number of staphylococcal bacteria included in the sample: ooo" is displayed. When it is determined that the bacteria included in a sample are bacteria which are not gram-positive cocci which are Staphylococcus, a message such as "Staphylococcal bacteria are not included in the sample" or "The number of staphylococcal bacteria included in the sample: 0" is displayed.

Next, a bacteria kind determination principle using lysostaphin will be described.

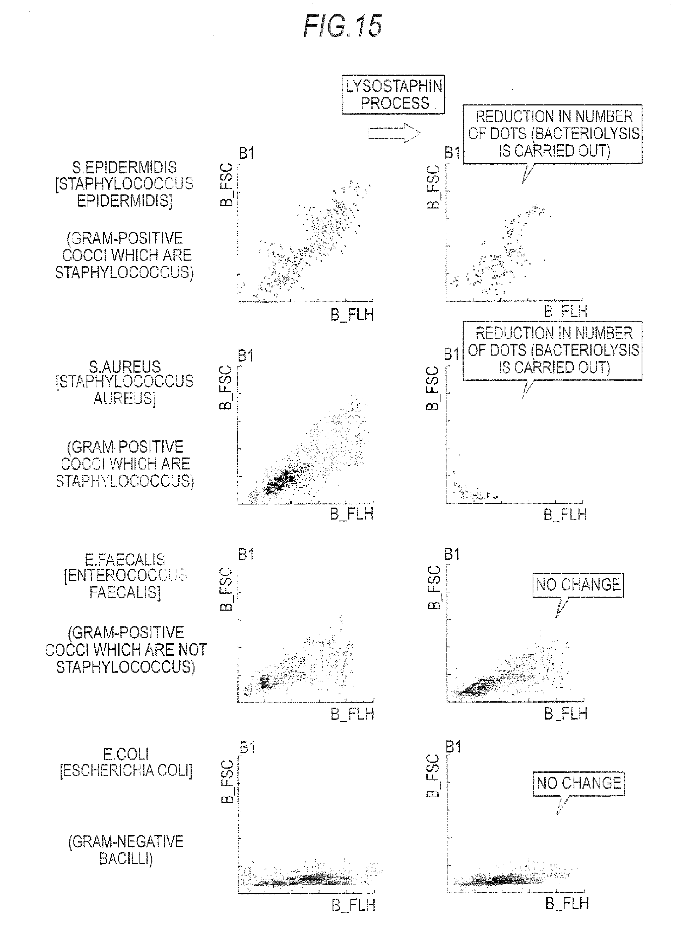

As shown in Fig. 15, as in the case of lysozyme shown in Fig. 11, scattergrams of a measurement specimen which is prepared from a sample, a diluent and a dye solution and a measurement specimen which is prepared from a sample, a diluent, a dye solution and lysostaphin are created by flow cytometry.

As shown in Fig. 15, regarding Enterococcus faecalis and Escherichia coli, a difference in the number of dots between the measurement result of the specimen for bacteria detection and the measurement result of the first specimen for bacteria determination cannot be shown. On the other hand, regarding Staphylococcus (Staphylococcus epidermidis and Staphylococcus aureus), the number of dots of the measurement result of the first specimen for bacteria determination is remarkably smaller than in the measurement result of the specimen for bacteria detection. That is, it is found that lysostaphin reacts only with Staphylococcus, not with Enterococcus faecalis and Escherichia coli.

In this manner, a sample is divided into two to prepare a measurement specimen by using one sample without the addition of lysostaphin and prepare a measurement specimen by using the other sample with the addition of lysostaphin. Whether or not the bacteria included in the sample are Staphylococcus can be determined on the basis of the measurement results of the two measurement specimens.

As the concentration of lysostaphin included in the first specimen for bacteria determination, it is desirable that in gram-positive cocci which are Staphylococcus such as Staphylococcus aureus and Staphylococcus epidermidis, a concentration is appropriately set at which a difference in the number of dots between the measurement result of the specimen for bacteria detection and the measurement result of the first specimen for bacteria determination can be recognized. The concentration of lysostaphin in the first specimen for bacteria determination is preferably in the range of 0.5 to 100 µg/mL. When the concentration of lysostaphin is in the above-mentioned range, the difference in the number of dots between the measurement result of the specimen for bacteria detection and the measurement result of the first specimen for bacteria determination can be easily recognized. The concentration of lysostaphin in the first specimen for bacteria determination is more preferably in the range of 0.5 to 2.5 µg/mL. At this time, the difference in the number of dots between the measurement result of the specimen for bacteria detection and the measurement result of the first specimen for bacteria determination can be more easily recognized, and thus the kind of bacteria included in the sample can be easily determined.

In this manner, a sample is divided into two aliquots to prepare a measurement specimen in one aliquot without addition of lysostaphin and prepare a measurement specimen in the other aliquot with addition of lysostaphin and it can be determined whether or not the bacteria included in the sample are Staphylococcus on the basis of the measurement results of the two measurement specimens.

In the second embodiment, as mentioned above, by determining the presence of reaction of bacteria by using lysostaphin which is a cell wall lytic enzyme specific for bacteria which are Staphylococcus, it can be easily determined whether or not the bacteria included in a sample are Staphylococcus.

In the second embodiment, as mentioned above, since lysostaphin reacts with gram-positive bacteria which are Staphylococcus, a tester can easily determine whether the bacteria included in a sample are gram-positive bacteria which are Staphylococcus or bacteria which are not Staphylococcus by using lysostaphin.

Other effects of the second embodiment are the same as in the above-mentioned first embodiment.

### (Third Embodiment)

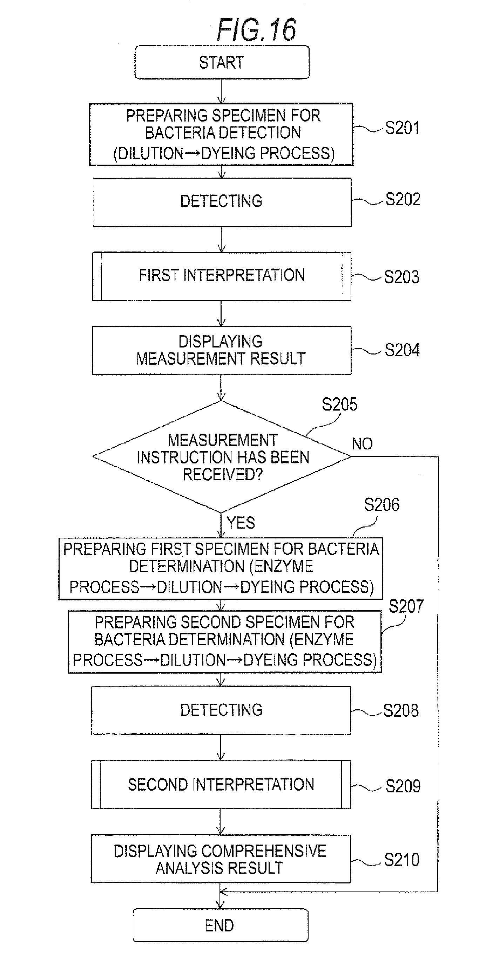

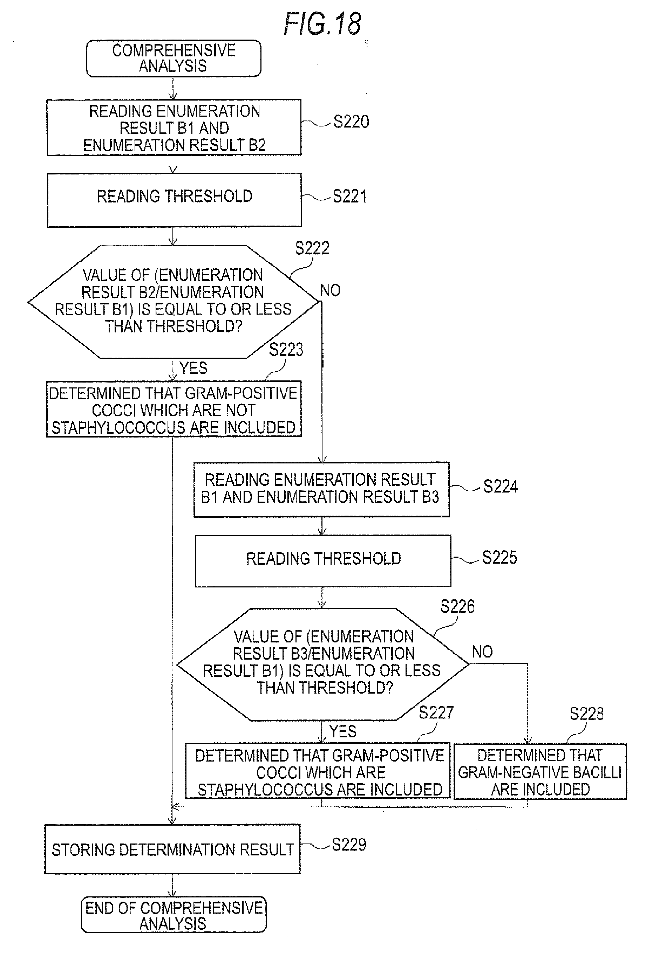

Next, a bacteria analysis apparatus 1 according to a third embodiment of the invention will be described with reference to Figs. 16 and 18. In the third embodiment, unlike the above-mentioned first and second embodiments, an example in which bacteria are analyzed by using two enzymes, that is, lysozyme and lysostaphin will be described. Since the structure of the bacteria analysis apparatus 1 according to the third embodiment is the same as in the above-mentioned first embodiment, except for an enzyme to be used and an interpretation process of the analysis section 3, the description of the structure of the bacteria analysis apparatus 1 will be omitted.

In an analysis operation according to the third embodiment, first, in Steps S201 to S204 of Fig. 16, the CPU 311 performs the same process as in Steps S1 to S4 of Fig. 5. A tester decides whether or not to perform second interpretation by referring to a first interpretation result screen as in the above-mentioned first embodiment. When the second interpretation is performed, the tester instructs the bacteria analysis apparatus 1 to perform the second interpretation by pressing a measurement instruction button (not shown) on the screen displayed on the display section 32 of the analysis section 3. In addition, in Step S205, the CPU 311 determines whether or not the measurement instruction button has been pressed. When the measurement instruction button has not been pressed, the analysis process of the bacteria analysis apparatus 1 ends. When the measurement instruction button has been pressed, the CPU 311 instructs the measurement section 2 to start the second interpretation.

When the instruction for the second interpretation is issued, in Steps S206 and S207, the control section 7 of the measurement section 2 controls the specimen preparation section 5 to prepare a first specimen for bacteria determination and a second specimen for bacteria determination. That is, the first specimen for bacteria determination is prepared by mixing a sample (urine), a diluent, a dye solution and an enzyme (lysozyme) and the second specimen for bacteria determination is prepared by mixing a sample (urine), a diluent, a dye solution and an enzyme (lysostaphin).

Next, in Step S208, the first specimen for bacteria determination and the second specimen for bacteria determination are subjected to the detection (measurement). The measurement result (data corresponding to forward-scattered light beams, data corresponding to side-scattered light beams and data corresponding to side fluorescent light beams) is transmitted to the analysis section 3.

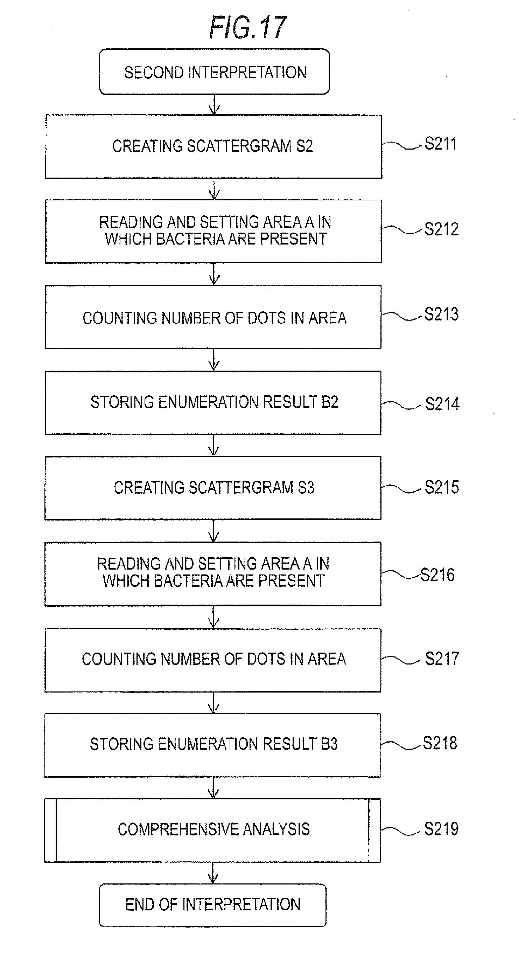

Next, in Step S209, the second interpretation process is performed by the analysis section 3. That is, in Step S211 of Fig. 17, a scattergram of the detection result of the first specimen for bacteria determination is created on the basis of the data corresponding to forward-scattered light beams of the first specimen for bacteria determination and the data corresponding to side fluorescent light beams. In addition, in Step S212, the area (see the area A of Fig. 7) set in the scattergram of the first interpretation is read and set in the scattergram of the first specimen for bacteria determination. In Step S213, the number of dots included in the area is counted in the scattergram of the first specimen for bacteria determination to obtain an enumeration result B2. The number of dots (enumeration result B2) is a value reflecting the number of bacteria included in the first specimen for bacteria determination. In Step S214, the enumeration result is stored in the hard disk 314.

In Steps S215 to S218, as in Steps S211 to S214, a scattergram of the detection result of the second specimen for bacteria determination is created, the number of dots included in the area is counted and an enumeration result B3 of the dots is stored.

After that, in Step S219, comprehensive analysis is performed (see Fig. 18).

In the comprehensive analysis, first, in Step S220 of Fig. 18, the enumeration result B1 of the specimen for bacteria detection and the enumeration result B2 of the first specimen for bacteria determination stored on the hard disk 314 are read. Then, a predetermined threshold which is set in advance is read from the hard disk 314 in Step S221.

In Step S222, the CPU 311 determines whether or not the value of (enumeration result B2/enumeration result B1) is equal to or less than the threshold. When the value of (enumeration result B2/enumeration result B1) is equal to or less than the threshold, the CPU 311 determines that the bacteria included in the sample are gram-positive cocci which are not Staphylococcus in Step S223. After that, the determination result is stored in Step S229.

When the value of (enumeration result B2/enumeration result B1) is greater than the threshold, the CPU 311 reads the enumeration result B1 of the specimen for bacteria detection and the enumeration result B3 of the second specimen for bacteria determination stored in the hard disk 314 in Step S224. Next, a predetermined threshold which is set in advance is read from the hard disk 314 in Step S225. in addition, in Step S226, it is determined whether or not the value of (enumeration result B3/enumeration result B1) is equal to or less than the threshold. When the value of (enumeration result B3/enumeration result B1) is equal to or less than the threshold, the CPU 311 determines that the bacteria included in the sample are gram-positive cocci which are Staphylococcus in Step S227. When the value of (enumeration result B3/enumeration result B1) is greater than the threshold, it is determined that the bacteria included in the sample are gram-negative bacilli in Step S228. Here, in the above-mentioned Step S222, it has been determined that the bacteria included in the sample are not Enterococcus faecalis (the bacteria included in the sample are Staphylococcus or gram-negative bacilli), and thus when the value of (enumeration result B3/enumeration result B1) is greater than the threshold (there are no effects of lysostaphin) in Step S228, it is estimated that the bacteria included in the sample are not Enterococcus faecalis and also not Staphylococcus. Accordingly, when the value of (enumeration result B3/enumeration result B1) is greater than the threshold in Step S228, it can be determined that the bacteria included in the sample are gram-negative bacilli.

In Step S229, after the storage of the determination result, the comprehensive analysis result is displayed on the comprehensive analysis result screen of the display section 32 in Step S210 (see Fig. 16). In the comprehensive analysis result screen, the scattergram of the detection result of the specimen for bacteria detection, the scattergram of the detection result of the first specimen for bacteria determination, the scattergram of the, detection result of the second specimen for bacteria determination and information based on the determination result in the above-mentioned Steps S223, S227 and S228 are displayed. The enumeration results B1, B2 and B3 are also displayed in the comprehensive analysis result screen. In greater detail, when it is determined that the bacteria included in the sample are gram-positive cocci which are not Staphylococcus, a message such as "Are Streptococcus included in the sample?", "Staphylococcal bacteria are not included in the sample. Streptococcus may be included in the sample" or "The number of staphylococcal bacteria included in the sample: 0" is displayed. When it is determined that the bacteria included in the sample are gram-positive cocci which are Staphylococcus, a message such as "Are staphylococcal bacteria included in the sample?", "Staphylococcal bacteria are included in the sample" or "The number of staphylococcal bacteria included in the sample: ooo" is displayed. When it is determined that the bacteria included in the sample are gram-negative bacilli, a message such as "Are Escherichia coli included in the sample?", "Staphylococcal bacteria are not included in the sample. Escherichia coli may be included in the sample" or "The number of staphylococcal bacteria included in the sample: 0" is displayed.

In the third embodiment, as mentioned above, by outputting information for supporting the determination of the kind of bacteria included in a sample on the basis of the detection result of a specimen for bacteria detection, the detection result of a first specimen for bacteria determination and the detection result of a second specimen for bacteria determination, the kind (bacteria which are Staphylococcus, gram-positive bacteria which are not Staphylococcus or gram-negative bacteria) of bacteria included in the sample can be more accurately estimated by using lysozyme and lysostaphin.

It should be considered that the disclosed embodiments are examples in all aspects but do not restrict the invention. The scope of the invention is defined with the claims, not with the above description of the embodiments, and all changes within the meaning and scope equivalent to the scope of the claims are included in the present invention.

For example, in the above-mentioned first to third embodiments, an example has been shown in which the kind of bacteria is determined by using lysozyme or lysostaphin. However, the invention is not limited thereto, and when there is an enzyme (cell membrane-digesting enzyme, cell wall lytic enzyme) reacting with certain bacteria, the determination may be performed by using the enzyme. By using such an enzyme, bacteria which are not the kinds of bacteria described in the above-mentioned first to third embodiments also can be determined.

In the above-mentioned first to third embodiments, an example has been described in which the sample is urine. However, the invention is not limited thereto, and another sample such as blood may be used if it can be measured by flow cytometry.

In the above-mentioned first to third embodiments, an example has been described in which the kind of bacteria is analyzed in the analysis section 3. However, the invention is not limited thereto, and the analysis may be performed by the control section 7 of the measurement section 2.

In the above-mentioned first to third embodiments, an example has been described in which a specimen for bacteria detection is prepared and measured by the bacteria analysis apparatus 1. However, the invention is not limited thereto. A specimen for bacteria detection may be prepared and measured by a different apparatus, the obtained measurement result may be transmitted to the analysis section 3 of the bacteria analysis apparatus 1 and comprehensive analysis may be performed by using the above measurement result and the measurement result of a specimen for bacteria determination. At this time, the measurement result can be provided from an external device, which is connected to the analysis section 3 by an electric communication line (which may be wired or wireless) to communicate therewith, through the electric communication line.

In the above-mentioned first to third embodiments, an example has been described in which one measurement specimen preparation section. 51 is provided. However, the invention is not limited thereto and a configuration may be provided in which at least two measurement specimen preparation sections are provided. For example, a configuration also may be provided in which two measurement specimen preparation sections are provided and prepare a specimen for bacteria detection and a first specimen for bacteria determination, respectively.

In the above-mentioned first to third embodiments, an example has been described in which one suction tube 8 is provided. However, the invention is not limited thereto and a configuration may be provided in which at least two suction tubes are provided. For example, a configuration also may be provided in which two measurement specimen preparation sections and two suction tubes are provided such that a specimen for bacteria detection and a first specimen for bacteria determination are dispensed to each of the measurement specimen preparation sections via each of the suction tubes.

In the above-mentioned first to third embodiments, an example has been described in which the degree of influence of an enzyme on the bacteria included in a sample is expressed by "enumeration result B2/enumeration result B1". However, the invention is not limited thereto. The degree of influence also may be expressed by, for example, "enumeration result B2-enumeration result B1" or "enumeration result B2/(enumeration result B1+enumeration result B2)".

In the above-mentioned first to third embodiments, an example has been described in which the display section 32 outputs the comprehensive analysis result. However, the invention is not limited thereto. The comprehensive analysis result may be printed on paper or may be transmitted to another apparatus.

In the above-mentioned first to third embodiments, an example has been described in which when a tester instructs the second interpretation using an enzyme after the first interpretation without using an enzyme, the second interpretation is performed. However, the invention is not limited thereto and a configuration also may be provided in which the first interpretation and the second interpretation are automatically performed without an instruction of the tester.

In the above-mentioned first to third embodiments, an example has been described in which a specimen for bacteria detection is prepared and measured, the necessity of the second interpretation is decided by the measurement result, and a first specimen for bacteria determination is prepared and measured when the second interpretation is required. However, the invention is not limited thereto and a configuration also may be provided in which both of a specimen for bacteria detection and a first specimen for bacteria determination are prepared and measured regardless of the necessity of the second interpretation.

### [Examples]

### First Example: Examination of Optimum Concentration of Lysozyme

In order to examine the optimum concentration of lysozyme in a measurement specimen, the number of bacteria in the measurement specimen in which the concentration of lysozyme had been changed was measured by using the fully automated urine formed element analyzer UF-1000i (manufactured by SYSMEX CORPORATION).
(1) Preparation of Measurement Specimen First, Enterococcus faecalis were added to bacterial broth and were left to cultivate overnight. With regard to the bacterial broth, a heart infusion medium (prepared by NISSUI PHARMACEUTICAL CO., LTD.) which is a liquid medium for general bacteria was used following the instruction manual, and after warming and melting, was then subjected to high-pressure steam sterilization.
   Next, the bacterial broth subjected to the overnight cultivation was added to the heart infusion medium so as to dilute it at a ratio of 1/500 and then was stirred. This broth was incubated at 35°C for 4 hours and was prepared as a Streptococcus faecalis specimen.
(2) Measurement of the Number of Bacteria
   Lysozyme (prepared by Wako Pure Chemical Industries, Ltd.) was added to the above-mentioned Streptococcus faecalis specimen such that a final concentration was 10 mg/mL, and then a reaction was carried out at 37°C for 5 minutes by a heat block.

The fully automated urine formed element analyzer UF-1000i (trade name) (manufactured by SYSMEX CORPORATION) was used to measure the number of bacteria included in the reacted specimen. UF II PACK-BAG (trade name) (prepared by SYSMEX CORPORATION) was used as a diluent and DF II SEARCH-BAG (trade name) (manufactured by SYSMEX CORPORATION) was used as a stain solution to measure the number of bacteria included in the specimen by following the instruction manual of DF-1000i.

Also in specimens to which lysozyme was added such that a final concentration was 0 mg/mL, 1 mg/mL, 2.5 mg/mL or 5 mg/mL, the number of bacteria was measured by using the same method as mentioned above.

### (Result)

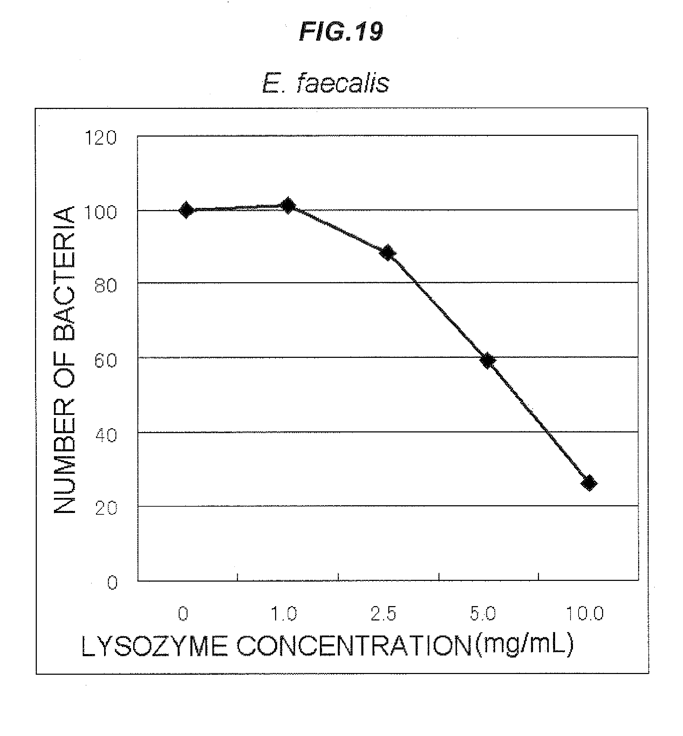

Fig. 19 shows a graph showing the number of bacteria at each concentration when the number of bacteria included in a specimen having a lysozyme concentration of 0 mg/m.L was set to 100.

From the graph of Fig. 19, it was found that when a final concentration of lysozyme was equal to or higher than 2. 5 mg/mL, the number of bacteria included in the specimen was smaller than in a specimen without the addition of lysozyme. In addition, it was found that the higher the final concentration of lysozyme, the smaller the number of bacteria included in the specimen. That is, it was found that the reaction between lysozyme and bacteria is dependent on the lysozyme concentration. From this, it was suggested that the higher the concentration of lysozyme included in a measurement specimen, the more easily the kind of bacteria can be determined.

### Second Example: Examination of Optimum Concentration of Lysostaphin

In order to examine the optimum concentration of lysostaphin in a measurement specimen, the number of bacteria in the measurement specimen in which the concentration of lysostaphin had been changed was measured by using the fully automated urine formed element analyzer UF-1000i (trade name) (manufactured by SYSMEX CORPORATION).

### (1) Preparation of Measurement Specimen

First, Staphylococcus aureus were added to bacterial broth and were left to cultivate overnight. With regard to the bacterial broth, a heart infusion medium (prepared by NISSUI PHARMACEUTICAL CO., LTD.) which is a liquid medium for general bacteria was used following the instruction manual, and after warming and melting, was then subjected to high-pressure steam sterilization.

Next, the bacterial broth subjected to the overnight cultivation was added to the heart infusion medium so as to dilute it at a ratio of 1/1000 and then was stirred. This broth was cultivated at 35°C for 4 hours and was prepared as a Staphylococcus aureus specimen.

In addition, in place of the above-mentioned Staphylococcus aureus, S. Sciuri was used to prepare a S. Sciuri specimen by using the same method as mentioned above.

### (2) Measurement of the Number of Bacteria

Lysostaphin (prepared by Wako Pure Chemical Industries, Ltd.) was added to the above-mentioned Staphylococcus aureus specimen such that a final concentration was 1.0 µg/mL, and then a reaction was carried out at 37°C for 5 minutes by a heat block.

The fully automated urine formed element analyzer UF-1000i (trade name) (manufactured by SYSMEX CORPORATION) was used to measure the number of bacteria included in the reacted specimen. UF II PACK-BAC (trade name) (prepared by SYSMEX CORPORATION) was used as a diluent and UF II SEARCH-BAG (trade name) (manufactured by SYSMEX CORPORATION) was used as a stain solution to measure the number of bacteria included in the specimen by following the instruction manual of UF-1000i.

Also in specimens to which lysostaphin was added such that a final concentration was 0 µg/mL, 0.5 µg/mL, 2.5 µg/mL, 5.0 µg/mL, 10.0 µg/mL or 100. 0 µg/mL, the number of bacteria was measured by using the same method as mentioned above.

Also in the S. Sciuri specimen, the measurement was performed by using the same method as mentioned above.

### (Result)

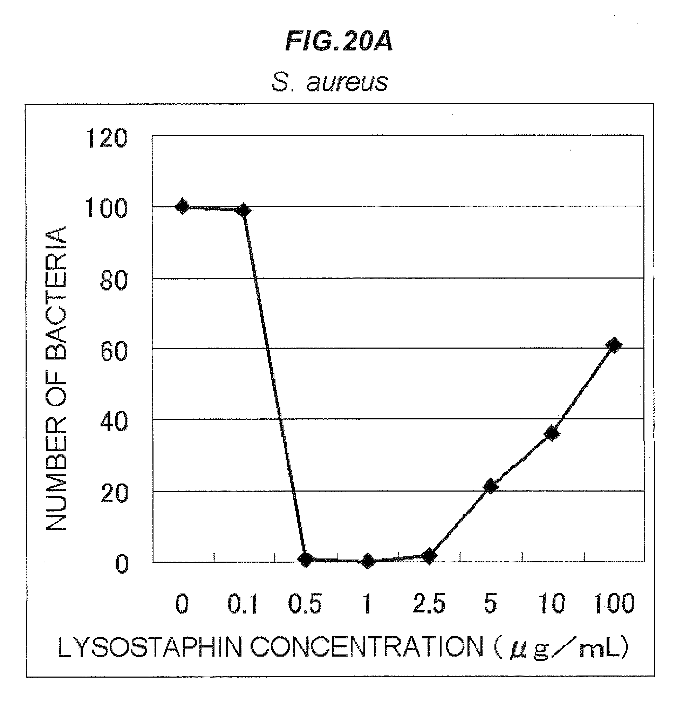

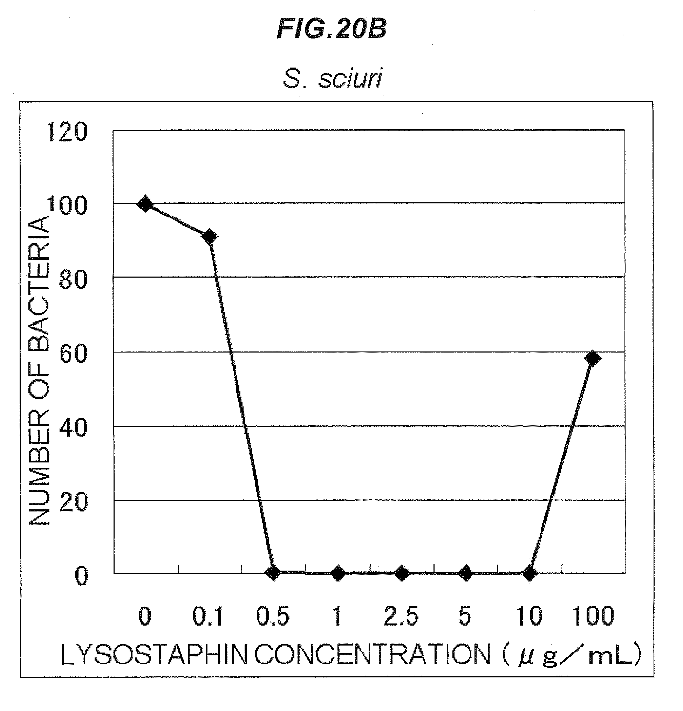

Fig. 20A and Fig. 20B shows a graph showing the number of bacteria at each concentration when the number of bacteria included in a specimen having a lysostaphin concentration of 0 µg/mL was set to 100.

From the graph of Fig. 20A and Fig. 20B, it was found that in any case of the Staphylococcus aureus specimen and the S. Sciuri specimen, the number of bacteria in the specimen having a final lysostaphin concentration of 0.5 to 100 µg/mL was smaller than in the case in which lysostaphin was not added. In addition, it was found that when a final concentration of lysostaphin was in the range of 0.5 to 2.5 µg/mL, the reactivity between lysostaphin and bacteria was the highest and the number of bacteria was nearly zero. That is, it was found that the reaction between lysostaphin and the Staphylococcus aureus specimen and S. Sciuri specimen is not dependent on the concentration in the same manner as the reaction of lysozyme and there is an optimum concentration for the reaction.

From this, it was suggested that when a final concentration of lysostaphin is in the range of 0.5 to 100 µg/mL, the kind of bacteria included in a measurement specimen can be easily determined, and further, when a final concentration of lysostaphin is in the range of 0.5 to 2.5 µg/mL, the kind of bacteria can be more easily determined.

## Claims

1. A bacteria analysis apparatus comprising:
a specimen preparation section for preparing a first measurement specimen from a sample by using a first enzyme;
a detecting section for detecting bacteria included in the first measurement specimen; and
an information processing section for outputting information for supporting determination of kind of bacteria included in the sample on the basis of the detection result of the first measurement specimen.

2. The apparatus of claim 1,
wherein the specimen preparation section prepares a second measurement specimen from the sample,
the detecting section detects bacteria included in the first measurement specimen and bacteria included in the second measurement specimen, and
the information processing section outputs information for supporting the determination of the kind of bacteria included in the sample on the basis of the detection result of the first measurement specimen and the detection result of the second measurement specimen.

3. The apparatus of claim 2,
wherein the information processing section obtains degree of influence of the first enzyme on the bacteria included in the sample on the basis of the detection result of the first measurement specimen and the detection result of the second measurement specimen, and outputs information for supporting the determination of the kind of bacteria included in the sample on the basis of the degree of influence.

4. The apparatus of claim 3,
wherein the information processing section obtains a value reflecting number of the bacteria included in the first measurement specimen and a value reflecting number of the bacteria included in the second measurement specimen on the basis of the detection result of the first measurement specimen and the detection result of the second measurement specimen, obtains the degree of influence of the first enzyme on the bacteria included in the sample on the basis of the value reflecting the number of bacteria of the first measurement specimen and the value reflecting the number of bacteria of the second measurement specimen, and outputs information for supporting the determination of the kind of bacteria included in the sample on the basis of the degree of influence.

5. The apparatus of claim 4,
wherein the information processing section creates a first scattergram relating to the bacteria included in the first measurement specimen and a second scattergram relating to the bacteria included in the second measurement specimen on the basis of the detection result of the first measurement specimen and the detection result of the second measurement specimen, and obtains the value reflecting the number of bacteria included in the first measurement specimen and the value reflecting the number of bacteria included in the second measurement specimen on the basis of the first scattergram and the second scattergram.

6. The apparatus of claim 3,
wherein the information processing section creates a first scattergram relating to the bacteria included in the first measurement specimen and a second scattergram relating to the bacteria included in the second measurement specimen on the basis of the detection result of the first measurement specimen and the detection result of the second measurement specimen, obtains the degree of influence of the first enzyme on the bacteria included in the sample on the basis of the pattern of the first scattergram and the pattern of the second scattergram, and outputs the information for supporting the determination of the kind of bacteria included in the sample on the basis of the degree of influence.

7. The apparatus of any one of claims 1 to 6,
wherein the information for supporting the determination of the kind of bacteria included in the sample at least includes a name of bacteria which may be included in the sample.

8. The apparatus of any one of claims 1 to 7, further comprising:
a display section,
wherein the information processing section performs a control operation so as to display information for supporting the determination of the kind of bacteria included in the sample on the display section.

9. The apparatus of any one of claims 1 to 8,
wherein the information for supporting the determination of the kind of bacteria included in the sample is information on whether or not the bacteria included in the sample are gram-positive bacteria.

10. The apparatus of any one of claims 1 to 9,
wherein the first enzyme is lysozyme, and the information for supporting the determination of the kind of bacteria included in the sample is information on whether or not the bacteria included in the sample are gram-positive bacteria which are not Staphylococcus or gram-negative bacteria and gram-positive bacteria which are Staphylococcus.

11. The apparatus of any one of claims 1 to 9,
wherein the first enzyme is lysostaphin, and the information for supporting the determination of the kind of bacteria included in the sample is information on whether the bacteria included in the sample are bacteria which are Staphylococcus or bacteria which are not Staphylococcus.

12. The apparatus of claim 2,
wherein the specimen preparation section further prepares a third measurement specimen from the sample by using a second enzyme,
the detecting section detects bacteria included in the first measurement specimen, bacteria included in the second measurement specimen and bacteria included in the third measurement specimen, and
the information processing section outputs information for supporting the determination of the kind of bacteria included in the sample on the basis of the detection result of the first measurement specimen, the detection result of the second measurement specimen and the detection result of the third measurement specimen.

13. The apparatus of claim 12,
wherein the first enzyme is lysozyme and the second enzyme is lysostaphin., and the information processing section, determines whether the bacteria included in the sample are bacteria which are Staphylococcus, gram-positive bacteria which are not Staphylococcus or gram-negative bacteria on the basis of the detection result of the first measurement specimen, the detection result of the second measurement specimen and the detection result of the third measurement specimen, and outputs information for supporting the determination of the kind of bacteria included in the sample on the basis of the determination result.

14. The apparatus of Claim 10 or claim 13,
wherein the concentration of lysozyme included in the measurement specimen is in the range of 2.5 to 20 mg/mL.

15. The apparatus of Claim 11 or claim 13,
wherein the concentration of lysostaphin included in the measurement specimen is in the range of 0.5 to 100 µg/mL.

16. A bacteria analysis method comprising:
preparing a first measurement specimen from a sample by using a first enzyme;
detecting bacteria included in the first measurement specimen; and
outputting information for supporting the determination of kind of bacteria included in the sample on the basis of the detection result of the first measurement specimen.

17. A computer program product comprising:
a computer readable medium; and
instructions, on the computer readable medium, adapted to enable a general purpose computer to perform operations, comprising:
obtaining the detection result of bacteria included in a first measurement specimen which is prepared from a sample by using a first enzyme; and
outputting information for supporting the determination of kind of bacteria included in the sample on the basis of the detection result of the bacteria of the first measurement specimen.
